# EUROPÄISCHE PATENTSCHRIFT

(11) **EP 4 208 126 B1**
(45) Veröffentlichungstag und Bekanntmachung des Hinweises auf die Patenterteilung: **09.10.2024**
(21) Anmeldenummer: 20775582.8
(22) Anmeldetag: 04.09.2020
(51) Int. Cl.: A61F 2/16

(54) **INJEKTOR FÜR INTRAOKULARLINSEN**
INJECTOR FOR INTRAOCULAR LENSES
INJECTEUR POUR LENTILLES INTRAOCULAIRES

(43) Veröffentlichungstag der Anmeldung: 12.07.2023
(73) Patentinhaber: OPHTHALMO Pro GmbH, 66386 Sankt Ingbert (DE)
(72) Erfinder: BAYER, Alexander, 40472 Düsseldorf (DE)
(74) Vertreter: Walther Bayer Faber Patentanwälte PartGmbB
(86) Internationale Anmeldenummer: PCT/EP2020/074776
(87) Internationale Veröffentlichungsnummer: WO 2022/048767

(56) Entgegenhaltungen:
- WO-A1-2019/195951
- US-A- 4 810 249
- US-A1- 2016 128 752
- US-B2- 8 105 332
- US-B2- 9 326 848

## Beschreibung

Die Erfindung betrifft einen Injektor zur Implantation einer Intraokularlinse in ein menschliches Auge, aufweisend einen Grundkörper, in den rückseitig ein länglich ausgebildetes Betätigungselement abschnittsweise hineinragt und das in einer Betätigungsachse beweglich geführt ist, und wobei eine Bewegung des Betätigungselementes in den Grundkörper wahlweise mittels einer Schubbetätigung entlang der Betätigungsachse oder mittels einer Schraubbetätigung um die Betätigungsachse herum erzeugbar ist, und wobei das Betätigungselement wenigstens auf einem Abschnitt ein Außengewinde aufweist, wobei am Grundkörper ein Stellelement angeordnet ist.

### STAND DER TECHNIK

Injektoren zur Implantation von Intraokularlinsen in ein menschliches Auge sind hinreichend bekannt. Eine Intraokularlinse kann hierfür entweder über eine Linsenkartusche mit einer in dieser eingebrachten Intraokularlinse kurz vor dem Einsetzen in das Auge in den Injektor eingebracht werden, um nach anschließender Zugabe eines Viskoelastikums die Intraokularlinse aus einer vorderseitigen Austriebsdüse in die hintere Augenkammer des Auges auszutreiben. Die Austriebsdüse bildet das offene Ende eines Linsenführungsteils des Injektors, der vorderseitig am Grundkörper angeordnet ist.

Im Grundkörper ist ein Kolben entlang der Betätigungsachse beweglich geführt, und der Kolben, der mit dem Betätigungselement in der Betätigungsachse voreinander liegend in Verbindung steht, kann bei einem Vorschub des Betätigungselementes und damit übertragen auf den Kolben in Richtung zur Austriebsdüse mit der Intraokularlinse in Kontakt gelangen und diese durch die Austriebsdüse austreiben.

Um den Kolben innerhalb des Grundkörpers des Injektors und weiter voranschreitend in Richtung zur Austriebsdüse auch durch das Linsenführungsteil hindurch kontrolliert zu bewegen, ist das Betätigungselement vorgesehen, das so in den Grundkörper hineinragt, dass bei einem Vorschub des Betätigungselementes in der Betätigungsachse der Kolben erst die Intraokularlinse kontaktiert und im weiteren Verlauf gemeinsam mit der Intraokularlinse in Richtung zur Austriebsdüse bewegt werden kann, bis die Intraokularlinse in die hintere Augenkammer ausgetrieben ist. Injektoren der neueren Bauart werden als Einweginjektoren verwendet und nach dem einmaligen Gebrauch und dem Austrieb der voreingelegten Intraokularlinse entsorgt. Diese Bauart der Injektoren wird allgemein als Pre-Load System bezeichnet.

Das Einschieben des Betätigungselementes in eine hintere Öffnung im Grundkörper erfolgt dabei in der Regel manuell durch den Bediener, also dem Ophthalmologen. Dabei sind Injektoren bekannt, die in einem ersten Betriebsmodus eine reine Schubbewegung ermöglichen, die manuell in das Betätigungselement eingebracht wird, und in einem zweiten Betriebsmodus eine Drehbewegung ermöglichen, die in das gleiche Betätigungselement eingebracht wird, um damit das Betätigungselement entlang der Betätigungsachse zu bewegen. Insofern sind Injektoren neuerer Bauart in der Lage, sowohl eine Schubbetätigung als auch eine Schraubbetätigung zu erlauben, wofür am Injektor eine entsprechende Vorrichtung zur Umschaltung des Betriebsmodus vorgesehen wird.

Beispielsweise offenbart die EP 3 476 375 A1 einen Injektor zur Implantation einer Intraokularlinse, und das Betätigungselement kann entweder durch ein rückseitiges Schieben oder durch eine Schraubbewegung in den Grundkörper eingetrieben werden, wobei die Schraubbewegung manuell in ein Stellelement eingeleitet werden kann, das eine direkte Gewindeverbindung mit einem Außengewinde des Betätigungselementes aufweist. Wird das Betätigungselement hingegen manuell in den Grundkörper hineingedrückt, so dreht das Stellelement frei mit. Wird das Betätigungselement wahlweise durch rückseitiges Drücken auf das Betätigungselement oder durch ein Drehen des Stellelementes in den Grundkörper hineingetrieben, so kann dieses in Kontakt mit dem Kolben die Intraokularlinse vorderseitig aus der Austriebsdüse austreiben.

Die WO 2019/195951 A1 offenbart eine weitere Ausgestaltung eines Injektors zur Implantation einer Intraokularlinse in ein menschliches Auge, aufweisend einen Grundkörper, in den rückseitig ein länglich ausgebildetes Betätigungselement abschnittsweise hineinragt und in einer Betätigungsachse beweglich geführt ist, und wobei eine Bewegung des Betätigungselementes in den Grundkörper wahlweise mittels einer Schubbetätigung des Betätigungselementes entlang der Betätigungsachse oder mittels einer Schraubbetätigung des Betätigungselementes um die Betätigungsachse erzeugbar ist, wofür das Betätigungselement wenigstens auf einem Abschnitt ein Außengewinde aufweist. Um zwischen der Schubbetätigung und der Schraubbetätigung des Betätigungselementes umzuschalten, sind zwei sich gegenüberliegende ein- und ausklappbare Flügelgriffe vorgesehen, wobei durch die ausgeklappte Position der Betriebsmodus auf eine Schubbetätigung und in einer eingeklappten Position auf eine Schraubbetätigung umgeschaltet werden kann. Im Betriebsmodus der Schubbetätigung können dabei die ein- und ausklappbaren Flügelgriffe als Fingergriffe dienen, sodass ein Operateur den Injektor an den Flügelgriffen zwischen Zeigefinger und Mittelfinger greifen kann, um schließlich mit dem Daumen rückseitig das Betätigungselement in den Grundkörper hineinzuschieben. Sind die Flügelgriffe eingeklappt, kann das Betätigungselement in den Grundkörper eingeschraubt werden, indem der Grundkörper in einer ersten Hand des Operateurs gehalten wird und das Betätigungselement mit der zweiten Hand des Operateurs eingeschraubt wird.

Ein weiterer Injektor ist aus der US 8,105,332 B2 bekannt, der ein am Grundkörper angeordnetes und um die Betätigungsachse drehbares Stellelement mit einem Durchgang aufweist, durch den sich ein Betätigungselement hindurch erstreckt. Dabei wirkt das Stellelement mit losen Kugeln zusammen, die mit einer Innenkontur des Stellelementes in Kontakt sind, wobei die Innenkontur einen veränderlichen Radius aufweist. Wird das Stellelement um die Betätigungsachse verdreht, so laufen die Kugeln gemäß einer Freigabeposition entweder außenseitig auf den Grundkörper auf oder diese werden gemäß einer Eingriffsposition radial nach innen in Öffnungen hinein gedrückt, die im Grundkörper eingebracht sind. In der Freigabeposition kann das Betätigungselement frei durch den Grundkörper hindurchlaufen und in der Eingriffsposition greifen die Kugeln in ein Außengewinde auf dem Betätigungselement ein, sodass dieses nun in den Grundköper eingeschraubt werden kann.

Aus der US 2016/0128752 A1 ist eine Misch- und Ausgabeeinrichtung für Knochenzement oder künstliche Knochenersatzmasse bekannt, die einen austauschbaren Druckkolben aufweist, der mit einem Außengewinde ausgeführt ist. Wird der Druckkolben in den Grundkörper eingedrückt, so wirkt dieser mit am Grundkörper angeordneten Rastklinken zusammen. So kann der Druckkolben in den beispielsweise mit Knochenzement oder mit künstlicher Knochenersatzmasse gefüllten Grundkörper eingedrückt werden, während dieser durch die Rastklinken an einem erneuten Herauslaufen gehindert ist. Die Rastklinken federn dabei beim Überlauf über das Gewinde radial auf, sodass der Druckkolben zwar in den Grundkörper eingedrückt aber nicht mehr herausgezogen werden kann. Um bei größeren notwendigen Kräften zum Eindrücken des Druckkolbens und zum Austreiben der Mischung beispielsweise aus Knochenzement oder aus Knochenersatzmasse den Druckkolben mit einer Schraubbewegung einzutreiben, kann eine Sperrhülse axial auf die Rastklinken aufgedrückt werden, sodass diese nicht mehr radial auffedern und insofern ein Innengewindegang bilden, in den der Druckkolben mittels des Außengewindes eingeschraubt werden kann.

Schließlich offenbart die US 4,810,249 A eine medizinische Spritze mit einem Kolben, der ein Außengewinde aufweist, und wahlweise kann der Kolben der Spritze mit einer an sich bekannten Druckbewegung in den Grundkörper der Spritze eingedrückt werden oder es wird eine am Grundkörper vorhandene Klinke aktiviert, die nach der Aktivierung einen Eingriff in das Außengewinde des Kolbens bildet, sodass der Kolben in den Grundkörper nicht mehr eingedrückt, sondern nur noch eingeschraubt werden kann.

Die Einstellung des Injektors entweder für die Schubbetätigung oder für die Schraubbetätigung erfolgt in der Regel durch eine assistierende Person, sodass anschließend der Ophthalmologe den Injektor mit dem voreingestellten Betriebsmodus anwendet, indem die Austriebsdüse am vorderen Linsenführungsteil in den Augapfel eingeführt wird, und indem anschließend die Betätigung des Betätigungselementes erfolgt, bis die Intraokularlinse in die hintere Augenkammer eingetrieben wurde.

### OFFENBARUNG DER ERFINDUNG

Aufgabe der Erfindung ist die Weiterbildung eines Injektors zur Implantation einer Intraokularlinse in ein menschliches Auge, wobei der Injektor eine möglichst einfache Umschaltbarkeit zwischen der Schubbetätigung und der Schraubbetätigung des Betätigungselementes zum Vortrieb der Intraokularlinse aufweisen soll. Insbesondere soll eine Umschaltung des Betriebsmodus zwischen der Schubbetätigung und der Schraubbetätigung so möglich sein, dass der Injektor im Übrigen auf gleiche Weise zu benutzen ist, wobei jedoch die Umschaltung zwischen der Schubbetätigung und der Schraubbetätigung unmittelbar vor der Benutzung des Injektors einstellbar sein sollte.

Diese Aufgabe wird ausgehend von einem Injektor gemäß dem Oberbegriff des Anspruchs 1 in Verbindung mit den kennzeichnenden Merkmalen gelöst. Vorteilhafte Weiterbildungen der Erfindung sind in den abhängigen Ansprüchen angegeben.

Die Erfindung schließt die technische Lehre ein, dass am Grundkörper wenigstens ein Federarm angeordnet ist, der an einer zum Außengewinde weisenden Innenfläche eine Gewindeeingriffsstruktur aufweist, wobei das Stellelement mit dem Federarm wechselwirkt und um die Betätigungsachse verdrehbar oder in der Betätigungsachse verschiebbar angeordnet ist, wobei und wobei das Stellelement mit dem Federarm über einen Wirkkontakt zusammenwirkt, wobei wenigstens eine am Wirkkontakt beteiligte Innenfläche am Stellelement und/oder eine Außenfläche am Federarm unter einem Winkel zur Betätigungsachse geneigt ausgebildet ist, sodass die Verdrehung oder die Verschiebung des Stellelementes eine elastische Verformung des Federarms zur Betätigungsachse hin und von der Betätigungsachse weg erzeugbar ist und sodass die Gewindeeingriffsstruktur mit dem Außengewinde wahlweise in Eingriff oder außer Eingriff bringbar ist.

Kerngedanke der Erfindung ist ein Stellelement, das manuell zwischen zwei Stellpositionen verdreht oder verschoben werden kann, und in einer ersten Stellposition bewirkt das Stellelement ein Eingreifen der Gewindeeingriffsstruktur am Federarm in das Außengewinde des Betätigungselementes für die Schraubbetätigung, und in der zweiten Stellposition gibt das Stellelement den Federarm mit der Gewindeeingriffsstruktur aus dem Außengewinde des Betätigungselementes frei, sodass dieses außer Eingriff mit dem Außengewinde am Betätigungselement gelangt und das Betätigungselement schließlich für die Schubbetätigung frei verschiebbar ist.

Wird also das Stellelement in die zweite Stellposition verdreht, so kann der Federarm oder können mehrere Federarme, insbesondere zwei sich gegenüberliegende Federarme, von selbst in Bezug auf die Betätigungsachse elastisch nach außen federn, sodass die Gewindeeingriffsstruktur das Außengewinde des Betätigungselementes freigibt. Die Wirkverbindung zwischen dem Stellelement und dem Federarm wird erfindungsgemäß durch einen Wirkkontakt erzeugt, der so ausgebildet ist, dass bei einer axialen Verlagerung des Stellelementes relativ zum Federarm eine Bewegung des Federarms erzeugt wird, die mit Bezug auf die Betätigungsachse in radialer Richtung erfolgt, nämlich dass der Federarm zur Betätigungsachse und damit zum Außengewinde des Betätigungselementes hin und von diesem wieder wegbewegt werden kann. Die axiale Richtung beschreit dabei eine sich in der Betätigungsachse erstreckende Richtung und eine radiale Richtung beschreibt eine sich senkrecht auf die Betätigungsachse stehende Richtung. Insofern stehen die axiale und die radiale Richtung im vorliegenden Sinne senkrecht aufeinander.

Um die Bewegung im Federarm durch eine Bewegung des Stellelementes zu erzeugen, weist wenigstens eine am Wirkkontakt beteiligte Fläche eine Neigung auf, die einen Winkel zum Verlauf der Betätigungsachse besitzt. Der Winkel kann dabei abgetragen werden zwischen der Betätigungsachse und einem Vektor, der in der beteiligten Fläche liegt, also der Innenfläche und/oder der Außenfläche, wobei der Vektor durch den Kontaktpunkt oder die Kontaktlinie zwischen der Innenfläche und der Außenfläche verläuft. Selbstverständlich kann die Innenfläche und/oder die Außenfläche des Stellelementes beziehungsweise des Federarms auch gewölbt oder auf sonstige Weise abweichend von einer Ebene ausgestaltet sein, ohne dabei vom erfindungsgemäßen Gedanken eines Wirkkontaktes unter einem Winkel abzuweichen.

Die Neigung der beteiligten Fläche kann die Innenfläche des Stellelementes sein, während die Außenfläche des Federarms gerade, das heißt in Verlaufsrichtung zur Betätigungsachse ohne einen Winkel verlaufend ausgebildet ist, während die Außenfläche entsprechend geneigt oder gewölbt ist.

Auf gleiche Weise kann auch die Außenfläche des Federarms gerade verlaufen, und die Innenfläche des Stellelementes ist geneigt oder gewölbt.

Selbstverständlich funktioniert das erfindungsgemäße Wirkprinzip zwischen dem Stellelement und dem Federarm besonders gut, wenn sowohl die Innenfläche als auch die Außenfläche eine Neigung oder eine Wölbung aufweisen, die insbesondere jeweils den gleichen Winkel zur Betätigungsachse einschließen. In jedem Falle der Ausgestaltung der Innenfläche und der Außenfläche entsteht jedoch ein Wirkkontakt, der unter einem Winkel relativ zur Betätigungsachse geneigt ist. Das Stellelement muss nicht einteilig ausgeführt sein, und dieses kann auch mehrteilig ausgebildet sein, sodass die Innenfläche auch an einem Teil des Stellelementes ausgebildet ist, das nicht von außen manuell bedient werden kann.

Mit weiterem Vorteil können die am Wirkkontakt beteiligten Flächen eine Beschichtung aufweisen, die ein besonders günstiges Abgleiten der Flächen aufeinander ermöglicht, sodass also die Innenfläche und/oder die Außenfläche die Beschichtung aufweist.

Beispielsweise ist das Stellelement als Schiebeelement ausgeführt und im oder am Grundkörper beweglich angeordnet. Die Innenfläche kann am Schiebeelement so ausgeführt sein, dass diese bei einer Verlagerung des Schiebeelementes am Grundkörper sich relativ zum Federarm verlagert, und das Prinzip des Wirkkontaktes unter einem Winkel zur Betätigungsachse kann insofern auch bei einem Schiebeelement genutzt werden. Im bevorzugten Fall ist das Stellelement ringförmig oder hülsenförmig ausgeführt und das Betätigungselement wird teilumfänglich oder vollumfänglich vom Stellelement umschlossen.

Ist das Stellelement ringförmig oder hülsenförmig ausgeführt, ist dieses bevorzugt so am Grundkörper angeordnet, dass die Rotationsachse des ringförmigen oder hülsenförmigen Stellelementes mit der Betätigungsachse zusammenfällt.

Dabei ist die Handhabung des Injektors mit einem verdrehbaren Stellelement in der Regel einfacher und angenehmer als das Schieben eines Schiebeelementes zwischen zwei Rastpositionen, wobei es jedoch auch denkbar ist, dass ein ringförmig oder hülsenförmig ausgestaltetes Stellelement ebenfalls in der Betätigungsachse am Grundkörper verschiebbar ist, dieses also zwischen zwei Rastpositionen verlagert werden kann.

Schließlich ist es denkbar, dass das Stellelement eine Schiebe-DrehBeweglichkeit in Anordnung am Grundkörper aufweist, woraus die am meisten bevorzugte Variante erzielt wird, nämlich wenn das Stellelement mit einer gewindeartigen Führung zwischen dem Stellelement und dem Grundkörper an letzterem verlagert werden kann. Wird sodann das Stellelement manuell verdreht, und erfolgt der Kontakt zwischen dem Stellelement und dem Grundkörper über eine entsprechende gewindeartige Führung, kann die axiale Verlagerung des Stellelementes in der Betätigungsachse auf diese Weise sehr komfortabel erzielt werden.

Insbesondere bildet das Stellelement einen manuell um die Betätigungsachse verdrehbaren Rotationskörper mit einem Innendurchgang, in den der wenigstens eine Federarm hineinragt und durch den sich das Betätigungselement hindurch erstreckt. So befindet sich zumindest abschnittsweise der Federarm in einem radial ausgebildeten Spalt, insbesondere Ringspalt, zwischen dem innenseitig verlaufenden Betätigungselement und dem außenseitigen Stellelement.

Mit noch weiterem Vorteil ist die Innenfläche im Innendurchgang des Stellelementes ausgebildet und/oder diese bildet einen um die Betätigungsachse umlaufenden Innenkegel. Wird also das Stellelement in der Betätigungsachse verlagert, so gelangt die Außenfläche am Federarm mit unterschiedlichen Abschnitten und damit mit unterschiedlichen Radien der Innenfläche im Stellelement in Kontakt, die durch eine Neigung aufgrund der Kegelstruktur den Federarm in Richtung zum Betätigungselement drücken oder den Abstand zum Betätigungselement wieder vergrößern, je nachdem, ob das Stellelement axial in eine erste oder in eine zweite Richtung bewegt wird. Diese Bewegung des Stellelementes in der Betätigungsachse kann dabei von einer Drehbewegung des Stellelementes um die Betätigungsachse überlagert werden.

Ein weiterer Vorteil wird erreicht, wenn der Grundkörper einen Aufnahmeabschnitt mit einem Durchgang aufweist, durch den sich das Betätigungselement hindurch erstreckt und wobei das Stellelement am oder auf dem Aufnahmeabschnitt zumindest abschnittsweise drehbeweglich oder schiebebeweglich aufgenommen oder angeordnet ist, und wobei sich der wenigstens eine Federarm in einen Bereich zwischen der Innenfläche des Stellelementes und dem Betätigungselement hinein erstreckt.

Das Stellelement ist entlang der Betätigungsachse axial beweglich und/oder um die Betätigungsachse drehbeweglich am oder auf dem Aufnahmeabschnitt geführt aufgenommen, wobei ein hinterer Abschnitt des Stellelementes über den Aufnahmeabschnitt hinausragt, in dem die unter dem Winkel geneigte Innenfläche am Stellelement ausgebildet ist, die mit dem wenigstens einen Federarm zusammenwirkt.

Schließlich können wenigstens ein Federarm, zwei sich gegenüberstehende Federarme, drei, vier oder mehr Federarme endseitig am oder innenseitig des Aufnahmeabschnittes an diesem oder am Grundkörper angeordnet sein, die innenseitig aus dem offenen Ende des hülsenförmigen Aufnahmeabschnittes herausragen und mit der Innenfläche des Stellelementes zusammenwirken.

Sind beispielsweise mehrere Federarme insbesondere über den Umfang gleich verteilt zwischen dem innenseitigen Betätigungselement und dem außenseitigen Stellelement angeordnet, kann eine Ausgestaltung nach Art einer Spannzange erreicht werden, wobei bei einer Anordnung von mehreren Federarmen um das Betätigungselement und dessen Außengewinde herum das sich ergebende Innengewinde aus den mehreren Gewindeeingriffsstrukturen der sich über dem Umfang gleich verteilt befindenden Federarme fast vollumfänglich ausgebildet werden.

Technisch möglich ist jedoch die erfindungsgemäße Ausgestaltung auch bereits mit einem einzigen Federarm an einer Umfangsposition, beispielsweise wenn gegenüberliegend dieses einzigen Federarms das Betätigungselement mit dem Außengewinde in einer dem Federarm gegenüberliegenden Lagerschale gehalten ist. Eine solche Variante wäre vorteilhaft, wenn das Stellelement als einfacher Schiebeschalter ausgebildet ist, der ebenfalls an nur einer Umfangsposition angeordnet ist.

Um eine Kulisse zu schaffen, aufgrund der bei einer manuell in das Stellelement eingeleiteten Drehbewegung das Stellelement sich zugleich in der Betätigungsachse bewegt, also der Drehbewegung eine Linearbewegung überlagert wird, ist es mit besonderen Vorteil möglich, im Außenumfang des Aufnahmeabschnittes wenigstens eine Nut einzubringen, wobei innenseitig im Stellelement wenigstens ein Zapfen ausgebildet ist, der in der Nut geführt ist. Mit dieser Nut-Zapfen-Verbindung wird eine Art Kulisse geschaffen, wobei die Nut vorzugsweise spiralförmig über einen Abschnitt oder den Vollumfang des Aufnahmeabschnittes ausgebildet ist. Beispielsweise kann das Stellelement um 90° oder 180° um die Betätigungsachse verdreht werden, sodass damit das Stellelement eine axiale Verlagerung in der Betätigungsachse erfährt, die so groß ist, dass über den Wirkkontakt unter dem Winkel der Außenfläche beziehungsweise der Innenfläche des Federarms beziehungsweise des Stellelementes der Federarm hinreichend weit in das Außengewinde des Betätigungselementes eingedrückt wird, das eine Schraubverbindung zwischen der Gewindeeingriffsstruktur des Federarms und dem Außengewinde des Betätigungselementes erzeugt werden kann. Mit besonderem Vorteil sind zwei sich gegenüberliegende oder drei auf dem Umfang gleich verteilte Nuten in einer schraubenförmigen Ausgestaltung im Außenumfang des Aufnahmeabschnittes eingebracht, in denen zugeordnete Zapfen verlaufen können, die innenseitig in der Innenfläche des Stellelementes ausgebildet sind. Dieser Bereich der Innenfläche des Stellelementes, der sich über den Aufnahmeabschnitt erstreckt, schließt sich dem Bereich an, in dem in der Innenfläche die schräge Fläche ausgestaltet ist, insbesondere der Innenkegel.

Insbesondere ergibt sich eine vorteilhafte Ausgestaltung, wenn das Stellelement mittels einer Gewindeverbindung auf dem Aufnahmeabschnitt angeordnet ist, sodass mit einer Verdrehung des Stellelementes um die Betätigungsachse das Stellelement zugleich eine Bewegung entlang der Betätigungsachse ausführt.

Schließlich kann der Grundkörper einen Flügelgriff aufweisen, wobei sich der Aufnahmeabschnitt rückseitig des Flügelgriffes als Fortsatz anschließt. Der Aufnahmeabschnitt, der Flügelgriff und der sonstige, übrige Grundkörper können einteilig und materialeinheitlich ausgestaltet und insbesondere im Spritzguss hergestellt sein.

Weiterhin ist es vorteilhaft, wenn der Injektor ein Aufnahmemittel für eine Linsenkartusche aufweist, wobei in die Linsenkartusche eine Intraokularlinse eingebracht ist und wobei die Linsenkartusche mit der Intraokularlinse in das Aufnahmemittel einsetzbar ist. Derartige Injektoren können insbesondere mehrfach verwendbar sein, sofern diese resterilisierbar sind. Jedoch kann auch ein mit einer Kartusche beladbarer Injektor als Wegwerf-Einwegartikel ausgeführt sein.

Der Injektor kann auch als sogenanntes Pre-Load-System ausgeführt sein, gemäß dem der Injektor eine im Grundkörper und/oder in einem vorderseitig am Grundkörper angeordneten Linsenführungsteil ausgebildete Aufnahmekammer aufweist, in der eine Intraokularlinse bereits herstellerseitig eingelegt ist, sodass der Injektor mit der eingelegten Intraokularlinse eine einzeln handhabbare und handelbare Einheit bildet. Der erfindungsgemäße Injektor kann insofern als Pre-Load-System oder als Kartuschensystem funktionieren.

Schließlich ist im Grundkörper ein Kolben aufgenommen, der vorteilhafterweise über einen Drehübertrager mit dem Betätigungselement axial verschiebbar ist. Insbesondere dann, wenn die Schraubbetätigung des Injektors eingeschaltet wird, muss das Betätigungselement die Drehbewegung ausführen, während der Kolben lediglich eine Linearbewegung in der Betätigungsachse ausführt, ohne sich dabei in der Betätigungsachse zu drehen.

Der Grundkörper ist vorteilhafterweise mit dem Aufnahmeabschnitt und mit dem Flügelgriff einteilig und aus nur einem Kunststoffkörper gebildet, wobei das vorderseitig am Grundkörper angeordnete Linsenführungsteil mit einer spitzenseitigen Austriebsdüse zum Austrieb der Intraokularlinse ausgebildet und an den Grundkörper insbesondere lösbar angeordnet ist.

### BEVORZUGTES AUSFÜHRUNGSBEISPIEL DER ERFINDUNG

Weitere, die Erfindung verbessernde Maßnahmen werden nachstehend gemeinsam mit der Beschreibung eines bevorzugten Ausführungsbeispiels der Erfindung anhand der Figuren näher dargestellt. Es zeigt:
- Figur 1: eine perspektivische Ansicht des Injektors zur Implantation einer Intraokularlinse in ein menschliches Auge,
- Figur 2: eine Explosionsdarstellung der wesentlichen Bestandteile des Injektors gemäß Figur 1,
- Figur 3: eine perspektivische Ansicht des Injektors mit einem aufgeschnitten dargestellten Stellelement, wobei das Stellelement in einer ersten Stellung angeordnet ist, in der der Injektor mit einer Schubbetätigung bedient werden kann,
- Figur 4: die perspektivische Ansicht des Injektors mit einem aufgeschnitten dargestellten Stellelement, wobei das Stellelement in einer zweiten Stellung angeordnet ist, in der der Injektor mit einer Schraubbetätigung bedient werden kann,
- Figur 5: eine weitere perspektivische Darstellung des Injektors mit einem aufgeschnitten gezeigten Stellelement, wobei das Stellelement durch eine axiale Verlagerung in einer ersten Stellung gezeigt ist, in der der Injektor mittels Schubbetätigung bedienbar ist, und
- Figur 6: die perspektivische Darstellung des Injektors mit dem aufgeschnitten gezeigten Stellelement, wobei das Stellelement durch eine axiale Verlagerung in einer zweiten Stellung gezeigt ist, in der der Injektor mittels Schraubbetätigung bedienbar ist.

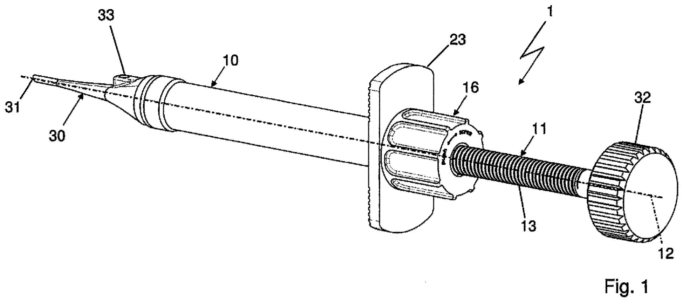

Figur 1 zeigt eine perspektivische Ansicht eines Injektors 1, der zur Implantation einer Intraokularlinse in ein menschliches Auge dient. Der Injektor 1 weist als wesentliches Strukturbauteil einen Grundkörper 10 auf, und in diesen ist rückseitig ein länglich ausgebildetes Betätigungselement 11 mit einem endseitigen Griffstück 32 teilweise hineingeführt. Das Betätigungselement 11 besitzt über seiner wesentlichen Länge einen Abschnitt mit einem Außengewinde 13, und das Betätigungselement 11 ist abschnittsweise rückseitig in den Grundkörper 10 so eingeführt, dass das Griffstück 32 an einem freien Ende des Betätigungselementes 11 ausgebildet ist.

Vorderseitig am Grundkörper 10 ist ein Linsenführungsteil 30 angeordnet, in dem in nicht näher gezeigter Weise eine Intraokularlinse aufgenommen ist. Der wesentliche Bestandteil des Grundkörpers 10 ist etwa zylinderförmig oder für die Handhabung ergonomisch für eine menschliche Hand ausgestaltet und weist eine längliche Erstreckung auf, und der Injektor 1 mit dem Grundkörper 10, mit dem vorderseitigen Linsenführungsteil 30 und mit dem rückseitig eingeführten Betätigungselement 11 erstreckt sich längs in der einer Betätigungsachse 12. Die Betätigungsachse 12 bildet dabei zugleich die Schubachse und die Rotationsachse für das wahlweise verschiebbare oder drehbare Betätigungselement 11.

Zur verbesserten Handhabung befindet sich am hinteren Teil des Grundkörpers 10 ein Flügelgriff 23, sodass der Injektor 1 mit dem Flügelgriff 23 zwischen dem Zeigefinger und dem Mittelfinger genommen werden kann, während mit dem Daumen rückseitig auf das Griffstück 32 gedrückt werden kann. Rückseitig zum Flügelgriff 23 schließt sich ein Stellelement 16 an, das um die Betätigungsachse 12 drehbar am Grundkörper 10 aufgenommen ist, und das Stellelement 16 besitzt einen Durchgang, durch den hindurch sich das Betätigungselement 11 erstreckt. Das Stellelement 16 ist dabei so am Grundkörper 10 eingerichtet, dass dieses keinen direkten Kontakt mit dem Betätigungselement 11 aufweist und mit diesem auch nicht unmittelbar zusammenwirkt, insbesondere nicht mit einer Gewindeverbindung.

Am Linsenführungsteil 30 ist eine Eingabeöffnung 33 gezeigt, durch die vor Inbetriebnahme des Injektors 1 ein Viskoelastikum eingegeben werden kann. Diese benetzt sodann die innenliegende Intraokularlinse und das innere Lumen insbesondere im Linsenführungsteil 30, um den Prozess des Austreibens der Intraokularlinse zu begünstigen beziehungsweise für die Intraokularlinse beschädigungsfrei zu ermöglichen. Der Austrieb der Intraokularlinse erfolgt schließlich über eine Austriebsdüse 31 an der freien Spitze des Linsenführungsteils 30.

Der Injektor 1 weist mit dem Stellelement 16 ein Mittel auf, mit dem für den Gebrauch des Injektors 1 zwischen einer Schubbetätigung und einer Schraubbetätigung umgeschaltet werden kann. Das Stellelement 16 ist für die Umschaltung zwischen der Schubbetätigung und der Schraubbetätigung im Ausführungsbeispiel gemäß Figur 1 um die Betätigungsachse 12 drehbar am Grundkörper 10 ausgebildet, wobei die Wechselwirkung des Stellelementes 16 mit dem Betätigungselement 11 in den weiteren Figuren näher dargestellt wird.

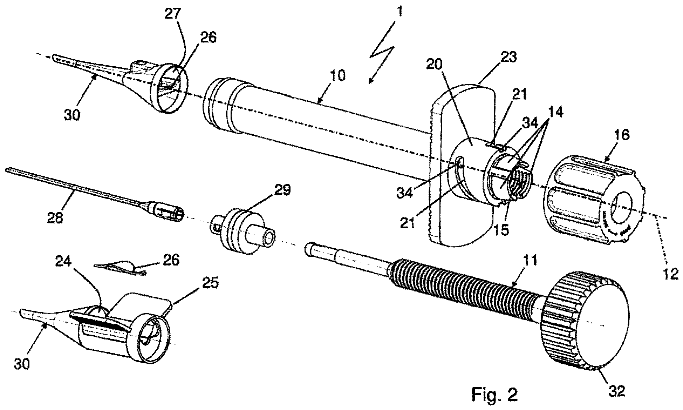

Figur 2 zeigt den Injektor 1 in einer Explosionsdarstellung, in der die wesentlichen Bestandteile des Injektors 1 dargestellt sind, wobei weitere Bestandteile des Injektors 1 vorhanden sind, die nicht dargestellt sind, da diese zur Darlegung der vorliegenden Erfindung nicht notwendig sind, die jedoch auch Bestandteil des erfindungsgemäßen Injektors 1 sein sollen, sodass die Abbildung nicht abschließend zu verstehen ist.

Die Darstellung zeigt den Grundkörper 10 vereinzelt vom Linsenführungsteil 30, und ein fluchtend mit der Betätigungsachse 12 dargestelltes Linsenführungsteil 30 ist mit einer Aufnahmekammer 27 ausgeführt, in der eine Intraokularlinse 24 eingelegt ist (sichtbar anhand einer der Haptiken), und ein unterseitig dargestelltes weiteres Linsenführungsteil 30 bildet eine alternative Ausgestaltung und weist ein Aufnahmemittel 24 zur Aufnahme einer Linsenkartusche 25 auf, in der die vereinzelt dargestellte Intraokularlinse 26 eingelegt ist. Wird die Intraokularlinse 26 in die Linsenkartusche 25 eingelegt und wird die Linsenkartusche 25 sodann in das Aufnahmemittel 24 eingelegt, kann die Intraokularlinse 26 mit einem Kolben 28 vorderseitig aus der Austriebsdüse 31 in die hintere Augenkammer des menschlichen Auges ausgetrieben werden. Ist der Injektor als Pre-Load System ausgeführt, kann das oberseitig dargestellte Linsenführungsteil 30 Anwendung finden, das eine Aufnahmekammer 27 aufweist, in der die Intraokularlinse 26 bereits herstellerseitig eingelegt ist.

Zum Austreiben der Intraokularlinse 26 führt der Kolben 28 eine Linearbewegung in der Betätigungsachse 12 aus, und wird das Betätigungselement 11 mit einer Schraubbetätigung in den Grundkörper 10 eingedreht, so dient ein Drehübertrager 29 dafür, dass die Rotationsbewegung des Betätigungselementes 11 nicht auf den Kolben 25 übertragen wird.

Mit anderen Worten: Wird nicht das Linsenführungsteil 30 mit der direkt in der Aufnahmekammer 27 aufgenommenen Intraokularlinse 26 genutzt, kann alternativ also das Linsenführungsteil 30 Anwendung finden, das mit dem Aufnahmemittel 24 ausgestattet ist, in das die Linsenkartusche 25 mit einer in dieser eingebrachten Intraokularlinse 26 eingesetzt werden kann. Dabei bildet das Linsenführungsteil 30 mit der Aufnahmekammer 27 das Pre-Load-System, gemäß dem der Injektor 1 mit der bereits werksseitig eingelegten Intraokularlinse 26 verkauft wird. Alternativ kann der Injektor 1 auch ohne vor-eingelegte Intraokularlinse 26 angeboten werden, wofür das Linsenführungsteil 30 das Aufnahmemittel 24 aufweist, in das die Linsenkartusche 25 mit der Intraokularlinse 26 erst kurz vor Gebrauch des Injektors 1 eingesetzt werden kann, was als ein sogenanntes Kartuschensystem bezeichnet wird. Das Aufnahmemittel 24 zur Aufnahme einer Linsenkartusche und/oder die Aufnahmekammer 27 zur direkten Aufnahme einer Intraokularlinse 26 können insofern alternativ auch im Grundkörper 10 eingerichtet sein, oder das Aufnahmemittel 24 oder die Aufnahmekammer 27 sind in einem Bereich zwischen dem Linsenführungsteil 30 und dem Grundkörper 10 ausgebildet, wenn das Linsenführungsteil 30 am Grundkörper 10 angeordnet wurde.

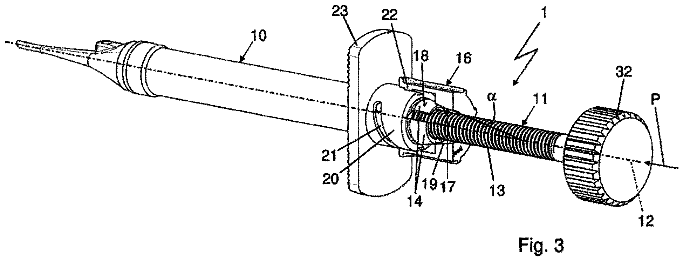

Hinter dem Flügelgriff 23 besitzt der Grundkörper 10 in Gestalt einer Anformung einen Aufnahmeabschnitt 20, auf den das Stellelement 16 um die Betätigungsachse 12 verdrehbar aufgebracht ist. Hierbei weist die Außenoberfläche des Aufnahmeabschnittes 20 eine Nut 21 auf, die spiralförmig über dem Aufnahmeabschnitt 20 in diesen eingebracht ist, sodass bei einer Verdrehung des Stellelementes 16 um die Betätigungsachse 12 das Stellelement 16 zugleich eine Bewegung in der Betätigungsachse 12 ausführt. An den Endabschnitten der Nut 21 können Rastmittel 34 eingebracht sein, und wird das Stellelement 16 in die jeweilige Endlage verdreht, gibt es für den Benutzer eine haptische Rückmeldung und das Stellelement 16 verharrt selbsttätig in der Endstellung. Innenseitig im Stellelement 16 sind zur Wirkverbindung mit der Nut 21 Zapfen eingebracht, die in der Nut 21 verlaufen und in der gezeigten Figur lediglich nicht dargestellt sind, sodass auf Fig. 3 verwiesen wird.

Der Aufnahmeabschnitt 20 ist hülsenförmig ausgeführt und insbesondere einteilig mit dem Grundkörper 10 rückseitig des Flügelgriffes 23 verbunden. Innerhalb des Aufnahmeabschnittes 20 sind beispielhaft drei Federarme 14 angeordnet, wobei die Federarme 14 entweder innenseitig im Aufnahmeabschnitt 20 oder rückseitig am Grundkörper 10 beziehungsweise am Flügelgriff 23 federelastisch angewurzelt sind. So können die Federarme 14 durch eine elastische Verformung in Richtung zur Betätigungsachse 12 nach innen oder von der Betätigungsachse 12 weg nach außen federnd bewegt werden. In einer nicht kraftbeaufschlagten Anordnung der Federarme 14 weisen diese einen Abstand zueinander bzw. zur Betätigungsachse 12 auf, gemäß dem das Außengewinde 13 auf dem Betätigungselement 11 mittig zwischen den Federarmen 14 frei bewegt werden kann. Der Auslieferzustand des Injektors 1 sollte daher in einer Stellung des Stellelementes 16 eingerichtet werden, in der dieses in der Stellposition für die Schubbetätigung steht, sodass die Federarme 14 nicht dauerhaft kraftbeaufschlagt sind und ihre Rückfederwirkung über eine längere Lagerzeit beibehalten.

Durch eine axiale Verlagerung des Stellelementes 16 in der Betätigungsachse 12 wird eine elastische Federbewegung der Federarme 14 in Richtung zur mittigen Betätigungsachse 12 erzeugt, insbesondere wenn das Stellelement 16 hin zum Flügelgriff 23 verlagert wird, vorzugsweise durch eine Verdrehung, sodass die Führung der Nut 21 die Verlagerung des Stellelementes 16 in der Betätigungsachse 12 in Richtung zum Flügelgriff 23 bewirkt. Durch den Kontakt mit der Innenfläche des Stellelementes 16 federn die Federarme 14 elastisch nach innen zur Betätigungsachse 12 hin ein, sodass eine Gewindeeingriffsstruktur 15 auf der Innenseite der Federarme 14 mit dem Außengewinde 13 des Betätigungselementes 11 in Eingriff gelangen kann, um sodann den Injektor 1 für die Schraubbetätigung einzurichten.

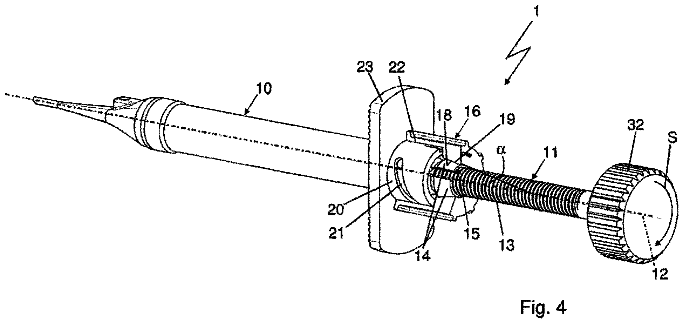

Die Figuren 3 und 4 zeigen den Injektor 1 einmal gemäß Figur 3 in einer Einstellung zur Schubbetätigung P und in Figur 4 in einer Einstellung zur Schraubbetätigung S.

Das hinter dem Flügelgriff 23 am Grundkörper 10 aufgenommene Stellelement 16 befindet sich in Figur 3 auf einer hinteren, von dem Flügelgriff 23 abgewandten Position, indem dieses mit Blick in der Betätigungsachse 12 aus Richtung des Griffstückes 32 entgegen dem Uhrzeigersinn verdreht wurde. Dabei verfahren Zapfen 22 auf der Innenseite des Stellelementes 16 in den Nuten 21 derart, dass das Stellelement 16 durch eine manuell eingebrachte Drehbewegung um die Betätigungsachse 12 axial verlagert wird, und in Figur 3 befindet sich das Stellelement 16 in der hinteren, die Federarme 14 öffnenden Position, sodass die Federarme 14 mit der innenseitigen Gewindeeingriffsstruktur 15 nicht in das Außengewinde 13 am Betätigungselement 11 eingreifen.

Um eine radiale Verlagerung der Federarme 14 hin zur Betätigungsachse 12 zu erzeugen, weist das Stellelement 16 einen Innenkegel 19 auf, der einen Teil der Innenfläche 17 des Stellelementes 16 bildet. Die Außenflächen 18 der Federarme 14 sind der Innenfläche 17 des Stellelementes 16 zugewandt, und die Außenfläche 18 weist gemäß dem Ausführungsbeispiel ebenso wie auch die Innenfläche 17 eine Neigung auf, die sich unter einem Winkel α zur Betätigungsachse 12 erstreckt. Verlagert sich also das Stellelement 16 in der Betätigungsachse 12, wird über die Neigung bzw. Schrägung der Innenfläche 17 und der Außenfläche 18, die in Kontakt zueinander stehen, die Position der Federarme 14 radial nach innen gedrückt oder radial wieder nach außen freigegeben, abhängig davon, ob das Stellelement 16 in Richtung zum Flügelgriff 23 oder vom Flügelgriff 23 weg verstellt wird.

So zeigt Figur 3 eine Position des Stellelementes 16 mit einem Abstand zum Flügelgriff 23, sodass durch den Kontakt der Federarme 14 mit der Innenfläche 17 auf einem größeren Durchmesser des Innenkegels 19 die Federarme 14 auffedern können, sodass der Injektor 1 in der Schubbetätigung P bedient werden kann.

Hingegen zeigt Figur 4 eine Position des Stellelementes 16, in der dieses an den Flügelgriff 23 angerückt ist, sodass die Außenflächen 18 der Federarme 14 mit einem Bereich der Innenfläche 17 des Stellelementes 16 in Kontakt sind, in der der Innenkegel 19 bereits einen kleineren Durchmesser aufweist, sodass die Gewindeeingriffsstruktur 15 in Eingriff mit dem Außengewinde 13 des Betätigungselementes 11 gelangt ist. So kann der Injektor 1 in der Schraubbetätigung S bedient werden.

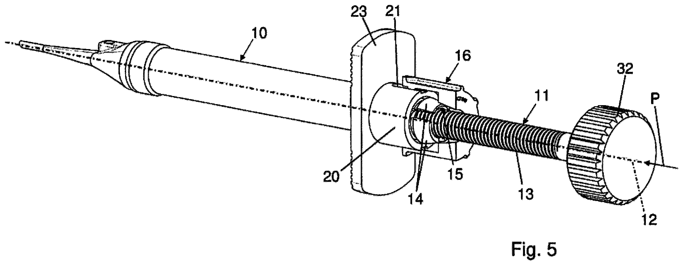

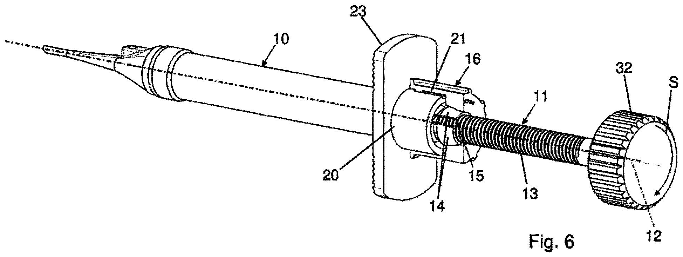

Die Figuren 5 und 6 zeigen ein weiteres Ausführungsbeispiel des Injektors 1 mit einer Anordnung des Stellelementes 16 auf einem Aufnahmeabschnitt 20 des Grundkörpers 10, und das Stellelement 16 wirkt bei einer axialen Verlagerung wie in vorstehend beschriebener Weise so auf die Federarme 14 ein, dass diese mit ihrer innenseitigen Gewindeeingriffsstruktur 15 mit dem Außengewinde 13 des Betätigungselementes 11 in und außer Wirkverbindung gebracht werden können.

Wird das Stellelement 16, wie in Figur 5 gezeigt, in einer Position angeordnet, in der dieses einen größeren Abstand zum Flügelgriff 23 aufweist, federn die Federarme 14 nach außen, und wird das Stellelement 16 in Richtung zum Flügelgriff 23 verlagert, so werden die Federarme 14 nach innen gedrückt, und die Gewindeeingriffsstruktur 15 auf der Innenseite der Federarme greifen in das Außengewinde 13 des Betätigungselemente 11 ein.

Figur 5 zeigt dabei die Position des Stellelementes 16 mit einem größeren Abstand zum Flügelgriff 23, sodass der Injektor 1 mit der Schubbetätigung P bedient werden kann, und in Figur 6 ist das Stellelement 16 in einer Position nahe am Flügelgriff 23 gezeigt, und die Federarme 14 werden nach innen gedrückt, sodass der Injektor 1 in der Schraubbetätigung S bedient werden kann.

Die Nut auf dem Aufnahmeabschnitt 20 verläuft dabei parallel mit der Betätigungsachse 12, sodass das Stellelement 16 gemäß diesem Ausführungsbeispiel nicht verdreht werden kann, und dieses kann vom Bediener lediglich axial verschoben werden zwischen den zwei vorstehend beschriebenen Endlagen, die insbesondere als Rastposition mit haptischer Rückmeldung ausgeführt sein können. Damit wird eine Ausführungsform verdeutlicht, gemäß der das Stellelement 16 auch als Schieber ausgeführt sein kann, wobei das Stellelement 16 auch nicht zwingend das Betätigungselement 11 vollumfänglich umschließen muss, und das Stellelement 16 kann abweichend von der Darstellung gemäß den Figuren 5 und 6 auch als an einer Umfangsposition angeordneter Schieber ausgestaltet sein.

Der Injektor 1 bietet damit den Vorteil, dass ein Operateur auch noch unmittelbar vor dem Eingriff in das menschliche Auge beispielsweise eigenhändig oder über eine Operationsassistenz entscheiden kann, ob er den Injektor 1 in der Schubbetätigung P oder in der Schraubbetätigung S bedienen will. Das Griffstück 32 ist lediglich beispielhaft dargestellt und kann auch alternativ ausgeführt sein, ebenso muss der Flügelgriff 23 nicht in einer sich in Querrichtung erstreckenden Weise ausgeführt sein, sondern dieser kann auch als Griffteller mit einem Kragen um die Betätigungsachse 12 ausgeführt sein. Das Ausführungsbeispiel gemäß den Figuren 5 und 6 kann dabei ebenfalls als Pre-Load-System oder als Kartuschensystem dienen.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht. Sämtliche aus den Ansprüchen, der Beschreibung oder den Zeichnungen hervorgehenden Merkmale und/oder Vorteile, einschließlich konstruktiver Einzelheiten oder räumlicher Anordnungen, können sowohl für sich als auch in den verschiedensten Kombinationen erfindungswesentlich sein.

## Patentansprüche

1. Injektor (1) zur Implantation einer Intraokularlinse, aufweisend einen Grundkörper (10), in den rückseitig ein länglich ausgebildetes Betätigungselement (11) abschnittsweise hineinragt und in einer Betätigungsachse (12) beweglich geführt ist, und wobei eine Bewegung des Betätigungselementes (11) in den Grundkörper (10) wahlweise mittels einer Schubbetätigung (P) entlang der Betätigungsachse (12) oder mittels einer Schraubbetätigung (S) um die Betätigungsachse (12) erzeugbar ist, und wobei das Betätigungselement (11) wenigstens auf einem Abschnitt ein Außengewinde (13) aufweist, wobei am Grundkörper (10) ein Stellelement (16) angeordnet ist,
**dadurch gekennzeichnet,**
**dass** am Grundkörper (10) wenigstens ein Federarm (14) angeordnet ist, der an einer zum Außengewinde (13) weisenden Innenfläche eine Gewindeeingriffsstruktur (15) aufweist, wobei das Stellelement (16) mit dem Federarm (14) wechselwirkt und um die Betätigungsachse (12) verdrehbar oder in der Betätigungsachse (12) verschiebbar ist, wobei das Stellelement (16) mit dem Federarm (14) über einen Wirkkontakt zusammenwirkt, wobei wenigstens eine am Wirkkontakt beteiligte Innenfläche (17) am Stellelement (16) und/oder eine Außenfläche (18) am Federarm (14) unter einem Winkel (α) zur Betätigungsachse (12) geneigt ausgebildet ist, sodass die Verdrehung oder die Verschiebung des Stellelementes (16) eine elastische Verformung des Federarms (14) zur Betätigungsachse (12) hin und von der Betätigungsachse (12) weg erzeugbar ist und sodass die Gewindeeingriffsstruktur (15) mit dem Außengewinde (13) wahlweise in Eingriff oder außer Eingriff bringbar ist.

2. Injektor (1) nach Anspruch 1,
**dadurch gekennzeichnet,**
**dass** das Stellelement (16) als Schiebeelement ausgeführt ist und im oder am Grundkörper (10) beweglich angeordnet ist und/oder dass das Stellelement (16) ringförmig oder hülsenförmig ausgeführt ist und das Betätigungselement (11) teilumfänglich oder vollumfänglich umschließt.

3. Injektor (1) nach einem der Ansprüche 1 oder 2,
**dadurch gekennzeichnet,**
**dass** das Stellelement (16) einen manuell um die Betätigungsachse (12) verdrehbaren Rotationskörper mit einem Innendurchgang bildet, in den der wenigstens eine Federarm (14) hineinragt und durch den sich das Betätigungselement (11) hindurch erstreckt.

4. Injektor (1) nach einem der vorgenannten Ansprüche,
**dadurch gekennzeichnet,**
**dass** die Innenfläche (17) im Innendurchgang des Stellelementes (16) ausgebildet ist und einen um die Betätigungsachse (12) umlaufenden Innenkegel (19) bildet, sodass bei einer axialen Verlagerung des Stellelementes (16) in der Betätigungsachse (12) die Außenfläche am Federarm (14) mit unterschiedlichen Abschnitten und damit mit unterschiedlichen Radien der Innenfläche (17) im Stellelement (16) in Kontakt kommt.

5. Injektor (1) nach einem der vorgenannten Ansprüche,
**dadurch gekennzeichnet,**
**dass** der Grundkörper (10) einen Aufnahmeabschnitt (20) mit einem Durchgang aufweist, durch den sich das Betätigungselement (11) hindurch erstreckt und wobei das Stellelement (16) am oder auf dem Aufnahmeabschnitt (20) beweglich aufgenommen ist, wobei sich der wenigstens eine Federarm (14) in einen Bereich zwischen der Innenfläche (17) des Stellelementes (16) und dem Betätigungselement (11) hinein erstreckt.

6. Injektor (1) nach einem der vorgenannten Ansprüche,
**dadurch gekennzeichnet,**
**dass** das Stellelement (16) entlang der Betätigungsachse (12) axial beweglich und/oder um die Betätigungsachse (12) drehbeweglich am oder auf dem Aufnahmeabschnitt (20) geführt ist.

7. Injektor (1) nach einem der vorgenannten Ansprüche,
**dadurch gekennzeichnet,**
**dass** wenigstens ein Federarm (14), zwei sich gegenüberstehende Federarme (14), drei, vier oder mehr Federarme (14) endseitig am oder innerhalb des Aufnahmeabschnittes (20) am Aufnahmeabschnitt (20) oder am Grundkörper (10) angeordnet ist bzw. sind.

8. Injektor (1) nach einem der vorgenannten Ansprüche,
**dadurch gekennzeichnet,**
**dass** im Außenumfang des Aufnahmeabschnittes (20) wenigstens eine Nut (21) eingebracht ist, wobei innenseitig im Stellelement (16) wenigstens ein Zapfen (22) ausgebildet ist, der in der Nut (21) geführt ist.

9. Injektor (1) nach einem der vorgenannten Ansprüche,
**dadurch gekennzeichnet,**
**dass** das Stellelement (16) mittels einer Gewindeverbindung auf dem Aufnahmeabschnitt (20) angeordnet ist, sodass mit einer Verdrehung des Stellelementes (16) um die Betätigungsachse (12) das Stellelement (16) eine Bewegung entlang der Betätigungsachse (12) ausführt.

10. Injektor (1) nach einem der vorgenannten Ansprüche,
**dadurch gekennzeichnet,**
**dass** der Grundkörper (10) einen Flügelgriff (23) aufweist, wobei sich der Aufnahmeabschnitt (20) rückseitig des Flügelgriffes (23) als Fortsatz anschließt.

11. Injektor (1) nach einem der vorgenannten Ansprüche,
**dadurch gekennzeichnet,**
**dass** der Injektor (1) ein Aufnahmemittel (24) für eine Linsenkartusche (25) aufweist, wobei in die Linsenkartusche (25) eine Intraokularlinse (26) eingebracht ist und wobei die Linsenkartusche (25) mit der Intraokularlinse (26) in das Aufnahmemittel (24) einsetzbar ist.

12. Injektor (1) nach einem der Ansprüche 1 bis 10,
**dadurch gekennzeichnet,**
**dass** der Injektor (1) eine im Grundkörper (10) und/oder in einem vorderseitig am Grundkörper (10) angeordneten Linsenführungsteil (30) ausgebildete Aufnahmekammer (27) aufweist, in der eine Intraokularlinse (26) eingelegt ist, sodass der Injektor (1) mit der eingelegten Intraokularlinse (26) eine einzeln handhabbare und handelbare Einheit bildet.

13. Injektor (1) nach einem der vorgenannten Ansprüche,
**dadurch gekennzeichnet,**
**dass** im Grundkörper (10) ein Kolben (28) aufgenommen ist, der über einen Drehübertrager (29) mit dem Betätigungselement (11) axial verschiebbar ist.

14. Injektor (1) nach einem der vorgenannten Ansprüche,
**dadurch gekennzeichnet,**
**dass** der Grundkörper (10) mit dem Aufnahmeabschnitt (20) und mit dem Flügelgriff (23) einteilig aus einem Kunststoffkörper gebildet ist, wobei das vorderseitig am Grundkörper (10) angeordnete Linsenführungsteil (30) mit einer spitzenseitigen Austriebsdüse (31) zum Austrieb der Intraokularlinse ausgebildet ist.

## Claims

1. Injector (1) for implantation of an intraocular lens (26), having a base body (10) and an elongated actuation element (11), which projects at least partly into a rear section of the base body (10) and which is movably guided in an actuation axis (12), and wherein a movement of the actuation element (11) into the base body (10) can be generated selectively by means of a push actuation (P) along the actuation axis (12) or by means of a screw actuation (S) about the actuation axis (12), and wherein the actuation element (11) features an external thread (13) at least on one section, and wherein to the base body (10) is arranged a switch element (16),
**characterized,**
**in that** at least one spring arm (14) is arranged at the base body (10), wherein the spring arm (14) has a thread engagement structure (15) on an inner surface facing the external thread (13), wherein the switch element (16) interacting with the spring arm (14) is arranged on the base body (10) so as to be rotatable about the actuation axis (12) or displaceable in the actuation axis (12), and wherein the switch element (16) interacts with the spring arm (14) via an operative contact, wherein at least one inner surface (17) of the switch element (16) involved in the operative contact and/or an outer surface (18) of the spring arm (14) involved in the operative contact is formed inclined at an angle (α) to the actuation axis (12), so that upon rotation or upon displacement of the switch element (16) an elastic deformation of the spring arm (14) towards the actuation axis (12) and away from the actuation axis (12) can be generated, whereby the thread engagement structure (15) can be selectively engaged or disengaged with the external thread (13).

2. Injector (1) according to claim 1,
**characterized,**
**in that** the switch element (16) is designed as a sliding element and is displaceable arranged in or on the base body (10) and/or in that the switch element (16) is designed ring-shaped or hull-shaped and encloses the actuation element (11) partially or completely.

3. Injector (1) according to one of claims 1 or 2,
**characterized,**
**in that** the switch element (16) forms a body of rotation, which can be rotated manually about the actuation axis (12) and which features an inner passage, into which the at least one spring arm (14) projects and through which the actuation element (11) extends.

4. Injector (1) according to one of the aforementioned claims,
**characterized,**
**in that** the inner surface (17) is formed in the inner passage of the switch element (16) and forms an inner cone (19) circumferential arranged about the actuation axis (12), so that when the actuating element (16) is axially displaced in the actuating axis (12), the outer surface on the spring arm (14) comes into contact with different sections and thus with different radii of the inner surface (17) in the actuating element (16).

5. Injector (1) according to one of the aforementioned claims,
**characterized,**
**in that** the base body (10) features a receiving section (20) with a passage, through which the actuation element (11) extends and wherein the actuation element (11) is movably received at or on the receiving section (20), so that the at least one spring arm (14) extends into an area between the inner surface (17) of the switch element (16) and the actuation element (11).

6. Injector (1) according to one of the aforementioned claims,
**characterized,**
**in that** the switch element (16) is received at or on the receiving section (20) in such a manner that the actuation element (11) is axially movable along the actuation axis (12) and/or is rotatable about the actuation axis (12).

7. Injector (1) according to one of the aforementioned claims,
**characterized,**
**in that** at least one spring arm (14), two spring arms (14) facing each other, three, four or more spring arms (14) are arranged on the rear end of the receiving section (20) or are arranged at the base body (10) in a position within the receiving section (20).

8. Injector (1) according to one of the aforementioned claims,
**characterized,**
**in that** at least one groove (21) is provided in the outer circumference of the receiving section (20), wherein at least one pin (22) is formed at the inside of the switch element (16) which pin (22) is guided in the groove (21).

9. Injector (1) according to one of the aforementioned claims,
**characterized,**
**in that** the switch element (16) is arranged on the receiving section (20) by means of a threaded connection, so that upon rotation of the switch element (16) about the actuation axis (12) the switch element (16) performs a movement along the actuation axis (12).

10. Injector (1) according to one of the aforementioned claims,
**characterized,**
**in that** the base body (10) features a wing handle (23), wherein the receiving section (20) adjoins the rear side of the wing handle (23) as an extension.

11. Injector (1) according to one of the aforementioned claims,
**characterized,**
**in that** the injector (1) features a receiving means (24) for a lens cartridge (25), wherein an intraocular lens (26) is inserted into the lens cartridge (25), and wherein the lens cartridge (25) with the intraocular lens (26) can be inserted into the receiving means (24).

12. Injector (1) according to one of claims 1 to 10,
**characterized,**
**in that** the injector (1) features a receiving chamber (27), which is formed in the base body (10) and/or in a lens guide section (30) arranged at the front end of the base body (10), and in which an intraocular lens (26) is inserted, so that the injector (1) forms an individually manageable and tradable unit with the inserted intraocular lens (26).

13. Injector (1) according to one of the aforementioned claims,
**characterized,**
**in that** a piston (28) is received in the base body (10) and can be displaced axially by the actuation element (11) via a rotary joint (29).

14. Injector (1) according to one of the aforementioned claims,
**characterized,**
**in that** the base body (10) is formed in one piece with the receiving section (20) and with the wing handle (23) made from a plastic body, wherein the lens guide section (30) with a tip-side ejection nozzle (31) for ejecting the intraocular lens (26) is arranged at the front end of the base body (10).

## Revendications

1. Injecteur (1) destiné à l'implantation d'une lentille intraoculaire, présentant un corps de base (10), dans lequel, sur le côté arrière, un élément d'actionnement (11) réalisé allongé dépasse en partie et est guidé mobile dans un axe d'actionnement (12), et dans lequel un déplacement de l'élément d'actionnement (11) dans le corps de base (10) peut être généré au choix au moyen d'un actionnement par coulissement (P) le long de l'axe d'actionnement (12) ou au moyen d'un actionnement par vissage (S) autour de l'axe d'actionnement (12), et dans lequel l'élément d'actionnement (11) présente, au moins sur une partie, un filetage extérieur (13), un élément de réglage (16) étant agencé sur le corps de base (10),
**caractérisé en ce que**
au moins un bras ressort (14) est agencé sur le corps de base (10), ledit bras ressort présentant, sur une face interne orientée vers le filetage extérieur (13), une structure de mise en prise par vissage (15), l'élément de réglage (16) interagissant avec le bras ressort (14) et étant rotatif autour de l'axe d'actionnement (12) ou coulissant dans l'axe d'actionnement (12), l'élément de réglage (16) coopérant avec le bras ressort (14) par le biais d'un contact fonctionnel, au moins une face interne (17) participant au contact fonctionnel étant réalisée sur l'élément de réglage (16) et/ou une face externe (18) étant réalisée sur le bras ressort (14) en formant un angle (α) par rapport à l'axe d'actionnement (12), de telle sorte que la rotation ou le coulissement de l'élément de réglage (16) peut provoquer une déformation élastique du bras ressort (14) vers l'axe d'actionnement (12) et à l'opposé de l'axe d'actionnement (12) et de telle sorte que la structure de mise en prise par vissage (15) peut au choix être amenée en prise avec le filetage extérieur (13) ou séparée de celui-ci.

2. Injecteur (1) selon la revendication 1,
**caractérisé en ce que**
l'élément de réglage (16) est conçu sous la forme d'un élément coulissant et est agencé mobile dans ou sur le corps de base (10) et/ou **en ce que** l'élément de réglage (16) est conçu en forme d'anneau ou en forme de douille et entoure l'élément d'actionnement (11) sur une partie ou sur la totalité de sa circonférence.

3. Injecteur (1) selon l'une des revendications 1 ou 2,
**caractérisé en ce que**
l'élément de réglage (16) forme un corps de révolution apte à être tourné manuellement autour de l'axe d'actionnement (12) et doté d'un passage intérieur, dans lequel l'au moins un bras ressort (14) dépasse et à travers lequel s'étend l'élément d'actionnement (11).

4. Injecteur (1) selon l'une des revendications précédentes,
**caractérisé en ce que**
la face interne (17) est réalisée dans le passage intérieur de l'élément de réglage (16) et forme un cône intérieur (19) périphérique autour de l'axe d'actionnement (12), de telle sorte que, lors d'un déplacement axial de l'élément de réglage (16) dans l'axe d'actionnement (12), la face externe située sur le bras ressort (14) vient en contact avec différentes parties et ainsi avec différents rayons de la face interne (17) à l'intérieur de l'élément de réglage (16).

5. Injecteur (1) selon l'une des revendications précédentes,
**caractérisé en ce que**
le corps de base (10) présente une partie de réception (20) dotée d'un passage à travers lequel s'étend l'élément d'actionnement (11) et dans lequel l'élément de réglage (16) est reçu de manière mobile contre ou sur la partie de réception (20), l'au moins un bras ressort (14) s'étendant dans une zone située entre la face interne (17) de l'élément de réglage (16) et l'élément d'actionnement (11).

6. Injecteur (1) selon l'une des revendications précédentes,
**caractérisé en ce que**
l'élément de réglage (16) est mobile axialement le long de l'axe d'actionnement (12) et/ou est guidé contre ou sur la partie de réception (20) de manière rotative autour de l'axe d'actionnement (12).

7. Injecteur (1) selon l'une des revendications précédentes,
**caractérisé en ce que**
au moins un bras ressort (14), deux bras ressorts (14) opposés l'un à l'autre, trois, quatre bras ressorts (14) ou plus est ou sont agencés côté extrémité sur ou à l'intérieur de la partie de réception (20) sur la partie de réception (20) ou sur le corps de base (10).

8. Injecteur (1) selon l'une des revendications précédentes,
**caractérisé en ce que**
au moins une rainure (21) est pratiquée dans la circonférence externe de la partie de réception (20), au moins un ergot (22), qui est guidé dans la rainure (21), étant réalisé sur le côté intérieur de l'élément de réglage (16).

9. Injecteur (1) selon l'une des revendications précédentes,
**caractérisé en ce que**
l'élément de réglage (16) est agencé sur la partie de réception (20) au moyen d'un assemblage vissé de telle sorte que, lors d'une rotation de l'élément de réglage (16) autour de l'axe d'actionnement (12), l'élément de réglage (16) exécute un déplacement le long de l'axe d'actionnement (12).

10. Injecteur (1) selon l'une des revendications précédentes,
**caractérisé en ce que**
le corps de base (10) présente une poignée papillon (23), la partie de réception (20) étant dans le prolongement de la poignée papillon (23) à l'arrière sous la forme d'un appendice.

11. Injecteur (1) selon l'une des revendications précédentes,
**caractérisé en ce que**
l'injecteur (1) présente un moyen de réception (24) pour une cartouche de lentille (25), une lentille intraoculaire (26) se trouvant dans la cartouche de lentille (25) et la cartouche de lentille (25) pouvant être introduite avec la lentille intraoculaire (26) dans le moyen de réception (24).

12. Injecteur (1) selon l'une des revendications 1 à 10,
**caractérisé en ce que**
l'injecteur (1) présente une chambre de réception (27) réalisée dans le corps de base (10) et/ou dans une pièce de guidage de lentille (30) agencée sur le corps de base (10) à l'avant, chambre de réception dans laquelle une lentille intraoculaire (26) est insérée, de telle sorte que l'injecteur (1) forme, avec la lentille intraoculaire (26) insérée, une unité pouvant être commercialisée et manipulée individuellement.

13. Injecteur (1) selon l'une des revendications précédentes,
**caractérisé en ce que**
un piston (28) est reçu dans le corps de base (10), ledit piston pouvant être déplacé axialement avec l'élément d'actionnement (11) par le biais d'un transmetteur rotatif (29).

14. Injecteur (1) selon l'une des revendications précédentes,
**caractérisé en ce que**
le corps de base (10) est formé avec la partie de réception (20) et avec la poignée papillon (23) d'un seul tenant en un corps en matière plastique, la pièce de guidage de lentille (30) agencée à l'avant sur le corps de base (10) étant réalisée avec une buse d'expulsion (31) du côté de la pointe, destinée à l'expulsion de la lentille intraoculaire.
